# EUROPÄISCHE PATENTANMELDUNG

(11) **EP 4 257 556 A2**
(43) Veröffentlichungstag der Anmeldung: **11.10.2023**
(21) Anmeldenummer: 23189316.5
(22) Anmeldetag: 13.03.2020
(51) Int. Cl.: C02F 1/28

(54) **PARTIKEL MIT BIOZIDER BESCHICHTUNG**

(30) Priorität: 15.03.2019 DE 102019106646
(62) Teilanmeldung aus: 20713212.7
(71) Anmelder: InstrAction GmbH, 69124 Heidelberg (DE)
(72) Erfinder: Welter, Martin, 69151 Neckargmünd (DE); Meyer, Christian, 68723 Schwetzingen (DE); Lungfiel, Christian, 65195 Wiesbaden (DE)
(74) Vertreter: Wetzel, Fritz

(57) **Zusammenfassung**

Die vorliegende Erfindung betrifft die Herstellung von Absorberharzen zur Bindung perfluorierter Tenside, wobei ein Verfahren die rein äußere Beschichtung von kommerziellen Ionenaustauscher bzw. die vollständige Beschichtung von porösen Polymerpartikeln mit einem Aminogruppen-haltigen Polymer und die anschließende Modifizierung des Polymers mit funktionalisierenden Liganden umfasst.

## Beschreibung

Die vorliegende Erfindung betrifft die Herstellung von Absorberharzen zur Bindung perfluorierter Tenside, wobei ein Verfahren die rein äußere Beschichtung von kommerziellen Ionenaustauscher bzw. die vollständige Beschichtung von porösen Polymerpartikeln mit einem Aminogruppen-haltigen Polymer und die anschließende Modifizierung des Polymers mit funktionalisierenden Liganden umfasst.

Laut den Berichten des World Economic Forum (2015), wird die Wasserkrise als das globale Risiko Nummer eins definiert, das die gesamte Weltbevölkerung betreffen wird.

Die Weltgesundheitsorganisation (WHO) und UNICEF betonen außerdem, dass 663 Millionen Menschen auf der Welt aktuell kein sauberes Trinkwasser zur Verfügung haben und 2,4 Milliarden Menschen Wasserquellen mit unzureichender Qualität verwenden (Stand 2015).

Die Gesamtsituation hat mehrere Ursachen, wobei hier ein kontinuierliches Bevölkerungswachstum, eine Verringerung der Wasserquellen aufgrund des Klimawandels und der globalen Erwärmung sowie der zunehmenden Verschmutzung von durch Industrieabfällen kontaminierten Wasserquellen angeführt werden kann. Daher ist es von entscheidender Bedeutung, die Trinkwasserqualität zu erhöhen und die Wasserverschmutzung zu verringern.

Trinkwasserquellen können durch verschiedene Schadstoffe kontaminiert sein. Dazu gehören Chemikalien, Bakterien, Mikroschadstoffe sowie Schwermetalle. Diese Verunreinigungen müssen aus gesundheitlichen Gründen vor dem Trinken aus dem Wasser entfernt werden.

Es gibt mehrere Möglichkeiten, Verunreinigungen zu entfernen, um unbedenkliches und sauberes Trinkwasser zu erhalten. Die verbreitetste Technik ist die mechanische Filtration, die in unterschiedlichen Techniken und Verfahren realisiert wird. Es kommt darauf an, dass Verunreinigungen mit Hilfe eines Filters, einer Membran oder dergleichen mit einer definierten Porengröße gefiltert werden, die kleiner ist als die Verunreinigung.

Die Umkehrosmose ist eine dieser Filtrationsmethoden, bei der der überwiegende Teil des zu filtrierenden Wassers mit hohen Betriebskosten (z.B. Strombedarf) als Abfall (ca. 90%) verworfen wird. Im Hinblick auf die Erschöpfung der Wasserquellen in der heutigen Welt ist es wichtig, kostengünstige und wassersparende Technologien für die Trinkwasserfiltration zu entwickeln.

Es ist mittlerweile offensichtlich, dass keine der am Markt verfügbaren Technologien alle Bereiche des möglichen Verunreinigungsspektrums alleine abdecken können. Dies gilt auch für die von der Firma instrAction GmbH angebotenen Harze, die beispielsweise "Chlor" nicht aus Wasser entfernen können. Daraus ergibt sich die Forderung nach einer intelligenten und neuartigen Kombination von bekannten Reinigungstechnologien (beispielsweise Aktivkohle für "Chlor") mit innovativen Absorber-Harzen für die Entfernung von Schwermetallen, Anionen von Elementsäuren bzw. elementigen Säuren, Mikroschadstoffe und Bakterien.

Die Produktivität wird, bei gegebenen Verunreinigungsprofil und gewünschten Abreicherungsraten, im Wesentlichen durch die notwendige Verweildauer im Absorberharzbett bestimmt. Dabei sind beispielsweise kleine Partikel aufgrund der kurzen Diffusionswege für die Kontaminanten zu bevorzugen, die allerdings auf der anderen Seite den Gegendruck in unerwünschter Weise erhöhen. Größere Partikel erfordern ggf. ein größeres Bett, da kleine Betten mit großen Partikeln nur schwer stabil gepackt werden können; auf der anderen Seite benötigen sie sehr wahrscheinlich eine längere Verweildauer, aufgrund der längeren Diffusionswege.

Bezüglich der am Markt verfügbaren Konkurrenztechnologien ist die Umkehrosmose (engl. Reverse Osmosis, RO) die am weitesten verbreitetste Technik. Gemäß der Marktstudie von Grand View Research (Market Research Reports & Consulting) von 2017 hat sie einem Marktanteil von ca. 44% im Bereich der heimischen Trinkwasseraufbereitung. Insgesamt wurden 2016 weltweit ca. 210 Millionen Einheiten verkauft.

Gravierender Nachteil der RO-Module ist ihre sehr schlechte Ausbeute. Nur ca. 10-20% des eingesetzten Wassers werden tatsächlich gereinigt und stehen dem Kunden zur Verfügung. Ein weiterer Nachteil ist der notwendige Einsatz von Strom für eine benötigte Pumpe und die Qualität des Wassers: RO-Systeme liefern reines Wassers, ohne lebenswichtige Salze, die dann wieder hinzugefügt werden müssen (z.B. Calcium und Magnesium).

Den Destillationsverfahren ist der Nachteil des extrem hohen Energieverbrauchs gemein. Zudem werden auch dort, wie bei der Umkehrosmose, die gesundheitsfördernden Elemente entfernt, so dass destilliertes Wasser entsteht, dass für den dauerhaften Konsum nicht geeignet ist, und es müssen wiederum wichtige Inhaltsstoffe wie Magnesiumsalze in einem darauffolgenden Schritt zugegeben werden.

Die Wasserreinigungsmaschinen, die mehrere Filtertechniken in separaten Einheiten/Kartuschen kombinieren, benötigen eine aufwändige Verrohrung mit entsprechenden Ventilen oder Konnektoren/Verbindern, die jedoch störanfällig sind und die Möglichkeit für Leckagen, etc. bieten. Zudem sind Verbindungen genau die Stellen, an denen Bakterien, etc. aufgrund der Strömungsverhältnisse besonders gute Möglichkeiten des Wachstums haben.

Ein bekanntes und auf dem Markt genutztes Filtermedium ist z. B. Aktivkohle, die als Schüttung von Partikeln in linear durchspülten Kartuschen oder als verpresster Hohlzylinder mit radialer Durchspülung eingesetzt wird.

Gleichzeitig geraten potenziell toxische Mikroschadstoffe mehr und mehr in den Focus des öffentlichen Interesses und der Fachwelt.

Dies gilt insbesondere für perfluorierte Tenside (z.B. Perfluoroktansäure, Perfluoroctansulfonsäure), die biologisch nicht oder kaum abbaubar sind und daher eine sehr hohe Persistenz haben ("Sachstandsbericht ADONA und perfluorierte Substanzen", Quelle: Bayerisches Landesamt für Gesundheit und Lebensmittelsicherheit).

Zur Entfernung von perfluorierten Tensiden aus Trinkwasser existieren eine Reihe sehr ineffizienter und teurer Methoden, wie etwa die Filtration über Aktivkohle, die für diese Art von Verunreinigungen nur eine sehr geringe Kapazität aufweist oder Membranverfahren, die zwar sauberes Trinkwasser bereitstellen, gleichzeitig aber große Abwassermengen produzieren, die die perfluorierten Tenside in angereicherter Form enthalten. Diese müssen zudem aufwendig entsorgt (in der Regel verbrannt) werden oder gelangen wieder ins Abwasser. Ein selektiver Absorber mit hoher Kapazität für perfluorierte Tenside gibt es unserem Kenntnisstand nach aktuell nicht.

Daraus ergibt sich die Aufgabe, bekannte Harze in einer Weise zu modifizieren und weiter zu entwickeln, dass sie neben den Schwermetallen und Bakterien auch perfluorierte Tenside aus Trinkwasser entfernen.

Die Aufgabe wurde gelöst wie in Anspruch 1 dargestellt. Vorteilhafte Ausgestaltungen sind Gegenstand der Unteransprüche.

Die Aufgabe wird gelöst durch ein Verfahren zur Herstellung von Partikeln zur Bindung perfluorierter Tenside, wobei das Verfahren die folgenden Schritte umfasst:
(a) Bereitstellen einer wässrigen Suspension enthaltend ein Polyamin, einen Vernetzer und ein poröses organisches oder anorganisches Trägermaterial in Partikelform bei einer Temperatur kleiner oder gleich 10 °C in einem Mischer zum Beschichten des Trägermaterials mit dem Polyamin;
(b) Vernetzen des Polyamins in den Poren des Trägermaterials und gleichzeitiges Entfernen von Wasser.

Im Sinne der Erfindung ist es bevorzugt, dass die Schritte a) und b) wenigstens einmal wiederholt werden.

Gemäß einer bevorzugten Ausführungsform des Verfahrens erfolgt das Vernetzen in einem Rührreaktor.

Als vorteilhaft hat sich herausgestellt, dass das Polyamin im nicht-entsalzten Zustand eingesetzt wird.

Gemäß einer weiteren Ausführungsform der Erfindung ist das organische Trägermaterial ausgewählt aus Polystyrol, Polymethacrylat oder Polyacrylat

Das organische Trägermaterial ist besonders bevorzugt ein Polystyrol.

Gemäß einer weiteren Ausführungsform ist das organische Trägerpolymer ein starker oder ein schwacher Anionenaustauscher, der nur auf seiner äußeren Oberfläche mit dem Polymer beschichtet ist. Als starker Anionenaustauscher werden solche organischen Polymere bezeichnet, die über Sulfonsäure-Gruppen verfügen. Schwache Anionenaustauscher sind Polymere, die Carbonsäure-Gruppen aufweisen.

Das Trägermaterial kann auch ein anorganisches Polymer sein, ausgewählt aus Kieselgel oder Hydroxyapatit.

Darüber hinaus ist es bevorzugt, wenn das Polyamin ein Polyvinylamin ist.

Gemäß einer weiteren Ausführungsform des erfindungsgemäßen Verfahrens ist es bevorzugt, dass das das Polyamin in den Poren und/oder auf der Oberfläche des beschichteten Partikels mit Glycidyl-2,2,3,3,4,4,5,5-octafluoropentylether 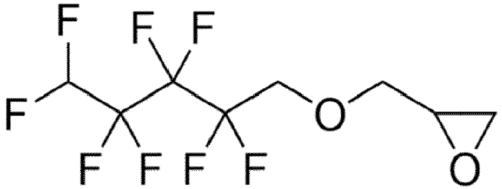 oder Perfluoroctansulfonsäure in der Seitenkette modifiziert wird.

Ein weiterer Gegenstand der Erfindung sind poröse Partikel, hergestellt nach einem Verfahren wie oben beschrieben.

Dabei ist bevorzugt, dass die porösen Partikel einen Substituenten 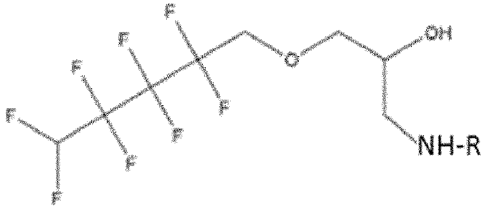 oder Perfluoroctansulfonsäure umfassen, wobei R das Polymer Polyvinylamin bedeutet.

Ein weiterer Gegenstand der Erfindung ist entsprechend auch die Verwendung der porösen Partikel wie oben beschrieben oder hergestellt nach dem oben beschriebenen Verfahren zur Bindung oder Entfernung von perfluorierten Tensiden aus Trinkwasser.

Von Harzen ist bekannt, dass sie Schwermetalle (WO2015EP01754, WO2016EP78787) und Bakterien (DE102017007273.6) aus Trinkwasser entfernen können. Dabei wird ein Amino-Polymer auf entsprechenden Trägern immobilisiert, zu einem stabilen dreidimensionalen Netzwerk mit einem bifunktionalen Vernetzer umgesetzt. Die Harze eigenen sich daher sowohl als Filter für Schwermetalle in Trinkwasserreinigungsanlagen als auch für "Polizeifilter" zur Entfernung von Schwermetallen am Ende einer Reinigungskaskade orthogonaler Methoden.

Im ersten Schritt der Harz-Herstellung wird das benötigte Beschichtungspolymer Polyvinylformamid erzeugt und mittels Natronlauge in einer polymeranalogen Reaktion zum Polyvinylamin (PVAm) hydrolysiert. Anschließend werden die Reagenzien und Abspaltungsprodukte durch eine zeit- und kostenintensive Crossflow-Filtration entfernt. Diese entsalzte Polymerlösung wird nun im zweiten Schritt für die Beschichtung des Trägermaterials eingesetzt.

Aufgrund von umwelttechnischen Aspekten (Reduktion des Energieverbrauchs und der Abwassermengen), sowie den Anforderungen des Marktes für Trinkwasserreinigungssysteme ergibt sich die Aufgabe, die Herstellprozesse zu vereinfachen, preiswerter und ressourcenschonender zu machen.

Bislang war nur bekannt, dass die sogenannten MetCap^{®} Partikel erfolgreich Bakterien aus Lösungen entfernen können (DE102017007273.6), die entweder auf Kieselgel basieren oder ohne Träger auskommen. Herstellung und Nachweis der Aktivität sind in DE102017007273.6 offenbart. Dort werden die Beschichtung von Kieselgelpartikeln (als Templat) mit nichtentsalztem Polymer und anschließender Auflösung der anorganischen Trägers und dessen antibakterielle Aktivität beschrieben.

Für Partikel, die auf einem organischen Träger, beispielsweise Polystyrol beruhen, war bislang keine entsprechende Aktivität bekannt. Diese Aktivität konnte nun auf dem nach dem neuen Verfahren hergestelltem Polystyrol-basierten Harz überraschenderweise festgestellt werden. Überraschend ist diese Beobachtung deshalb, da Polystyrole üblicherweise zur ausgeprägten Biofilmbildung neigen und keinesfalls Bakterien entfernen. Offenkundig sorgt die neuartige Beschichtung dafür, dass die Bakterien auf den Polystyrol-Oberflächen nicht wachsen, sondern vielmehr aus der Matrix, hier Trinkwasser, gebunden werden.

Aus den genannten Gründen ergeben sich eine Reihe von Aufgaben zur Entwicklung und Weiterentwicklung der bekannten Harze mit folgenden Zielsetzungen:
Vereinfachung des aktuellen Herstellverfahrens zur Produktion der instrAction MetCap und BacCap-Harze, Reduktion des Abfallstroms, die Erweiterung des Produktspektrums durch Kombination von Ionenaustauscher-Eigenschaften zur Herstellung von Absorberharzen zur Bindung von perfluorierten Tensiden, die ein zunehmendes Problem in der Trinkwasseraufbereitung darstellen. Des Weiteren wäre eine Erweiterung des Produktspektrums auf organische Träger, beispielsweise Polystyrol, von großem Vorteil, da diese preiswert in unterschiedlichen Spezifikationen hinsichtlich Partikelgröße und Porenweite erhältlich sind. Polystyrole haben gute mechanische Eigenschaften für die Anwendung und sind am Markt gut eingeführt.

Die Vereinfachung des Herstellprozesses unter Verwendung Polystyrol-basierter Harze wird durch Verzicht auf die Entsalzung des Polymerhydrolysats sowie von weiteren Prozessänderungen erreicht, die insbesondere die Zugabe und Trocknung der Trägerpolymere zu der Polymerlösung betreffen.

Überraschenderweise ist es möglich, MetCap^{®} und BacCap^{®} Harze ohne vorherige Entsalzung der Polymerlösung durch Immobilisierung auf porösen Polystyrol-Partikeln herzustellen. Dies ist umso überraschender, als in früheren Studien eine deutliche Abhängigkeit der Abscheidungs- oder Immobilisierungsrate des Polymers auf dem porösen Träger vom Salzgehalt des Polymerhydrolysats gefunden wurde.

Durch Anpassungen im Beschichtungsprozess (z.B. Mehrfachbeschichtung, Trocknung im Loedige-Pflugscharmischer, Einführung neuer Waschstrategien) konnte auf den aufwendigen und kostspieligen Prozessschritt der Entsalzung des Polymerhydrolysats verzichtet werden, ohne Einschränkungen in der Leistungskraft der Produkte hinnehmen zu müssen.

Zusammenfassend kann man sagen, dass die Änderung des Herstellungsprozesses, insbesondere der Verzicht auf die Entsalzung durch Membranfiltration und die Erweiterung auf organische Trägermaterialien entscheidende Vorteile bringt.

Der Polymergehalt wird nun durch die Ansatzberechnung bei der Polymerisation bestimmt. Die Beschichtung und Vorvernetzung mit Ethylenglycoldiglycidylether im Lödige Vakuumschaufeltrockner funktioniert zur Überraschung der Autoren genauso wie mit der PVAm-Polymerlösung, die keine Salze enthält. Die enthaltenen Salze werden dann bei der Herstellung der Suspension für die Nachvernetzung teilweise herausgelöst. Nachdem das Kieselgel des Trägers mit Hilfe der Natronlauge in Lösung gebracht wurde, werden alle Salze (Silicate, Formiate, Chloride, etc.) aus dem vernetzten, rein organischen Templatmaterial herausgespült. Das so erhaltene BacCap^{®} T beziehungsweise MetCap^{®} T Material hat die gleichen Eigenschaften, wie die Absorberharze, die nach dem Verfahren mit dem entsalzten PVA-Polymer hergestellt wurden. Dieses ist die erste Verbesserung in dem Prozess, die sehr überraschend kommt, weil die bisherigen, auch durch Literatur-Daten belegte, Annahme vorherrschte, der Volumenbedarf der hochkonzentrierten Salze in der Polymerlösung verhindere eine effektive und vollständige Füllung der Partikel mit Polymer, alleine durch seine Raumbeanspruchung.

Das zweite Verfahren betrifft die Beschichtung von kommerziellen starken oder schwachen Ionenaustauschern mit einer anti-bakteriellen PVA-Polymerhülle.

Kommerzielle Ionenaustauscher, speziell die hier verwendeten Kationenaustauscher, weisen in der Regel Säuregruppen auf die kovalent an den polymeren Träger (z.B. Polystyrol, Acrylate etc.) gebunden sind. Bei den Säuregruppen handelt es sich um Carbonsäuren bzw. Carboxylate bei schwachen Ionenaustauschern oder Sulfonsäuren bzw. Sulfonate bei starken Ionenaustauschern. In der Enthärtung von Trinkwasser werden beide Typen verwendet.

Um diese Ionenaustauscher mit anti-bakteriellen Eigenschaften auszustatten und gleichzeitig ihre Enthärtungskapazität nicht signifikant zu reduzieren, wird lediglich eine äußere Beschichtung der Partikel angestrebt, ohne die Säure-Gruppen in den Poren der Partikel, wo sich der überwiegenden Teil der Kapazitäts-tragenden Säure-Gruppen befindet, zu modifizieren.

Erreicht wird dieses Ziel durch Verwendung eines entsprechenden Polymers, das aufgrund seiner Größe und seines hydrodynamischen Radius nicht in die Poren der Ionenaustauscher-Partikel eindringen kann. Die Porengrößen bei kommerziellen Ionenaustauschern liegen im Bereich von 20 nm bis 100 nm. Für Polymere der Größe 10.000 - 20.000 g/mol sind diese Poren unzugänglich.

Bei diesem Vorgehen werden in einer bevorzugten Ausführungsform lediglich die äußeren 2-25% des Partikels gemessen am Radius des Partikel beschichte. Stärker bevorzugt werden nur die äußeren 2 - 10% des Partikels gemessen am Radius des Partikel beschichtet. Am stärksten bevorzugt werden nur die äußeren 2 - 5% des Partikels gemessen am Radius beschichtet.

Das Polymer enthält nach der Hydrolyse der Amid-Gruppen des Polyvinylamins mit Natronlauge und dem anschließenden Abstumpfen mit Salzsäure ca. 15-25 Gew% Salz in Form von Natriumformiat und Kochsalz. Der Polymergehalt der wässrigen Lösung entspricht im Fall des nicht entsalzten Polymers 9-13 Gew%.

In den bisherigen Verfahren wurden die Salze durch Umkehrosmose aufwendig entfernt, und das Polymer mit einem Salzanteil von weniger als 2,5 Gew% eingesetzt. Das neue Verfahren erlaubt den Verzicht auf diesen aufwendigen und teuren Entsalzungsschritt. Es ist daher bevorzugt mit dem neuen Verfahren das Polymer teilentsalzt mit einem Salzanteil von 2,5-15 Gew% einzusetzen. Stärker bevorzugt ist es, ein teilweise entsalztes Polymer mit einem Salzanteil von 10-15 Gew% einzusetzen. Am stärksten bevorzugt ist es, ein nicht entsalztes Polymer mit einem Salzanteil von 15-25 Gew% einzusetzen.

### Beispiel 1

1712 g feuchtes Trägermaterial Ionenaustauscher Lewatit S1567 werden direkt in einem Pflugscharmischer VT5 der Firma Lödige gefördert. Dann wird der Ionenaustaucher für 60 min bei 80 C getrocknet. Durch Wiegen des getrockneten Ionenaustauschers wird der Feuchtigkeitsverlust bestimmt. Es wurden 380 g Wasser entfernt. Die Produkttemperatur im Trockner wird auf 10 °C eingestellt. Der Mischer wird mit 180 Umdrehungen pro Minute betrieben. Nach dem die Produktemperatur in der Mischtrommel 10°C erreicht hat, werden 225 g des Ionenaustauschers auf 10°C gekühlte nicht entsalzte Polyvinylaminlösung Lot.: PC 18007 (Polymergehalt 10%) und 1 g Ethylen-Glykol-Di-Glycidyl-ether (EGDGE) [2224-15-9] in einem Gefäß eingewogen und VE- Wasser zugegeben bis ein Volumen von insgesamt 350 ml erreicht wurde. Die Mischung wird innerhalb von 10 min in dem Mischer gegeben und für 1 h bei 10 °C gemischt. Anschließend wird das Polymeradsorbat bei 80 °C und vermindertem Druck von 50 mbar für 2 h vernetzt. Der polymerbeschichtete Ionenaustauscher wurde dann auf Raumtemperatur heruntergekühlt.

Die Partikel werden dann auf geeignete Filternutsche überführt und mit folgenden Lösungsmitteln gewaschen (BV = Bettvolumen): 3 BV 0,1 M NaOH, 3 BV VE-Wasser, 3 BV 0,2 M HCl, 3 BV Wasser, 6 BV 0,1 M NaOH, 6 BV VE-Wasser. Das Produkt BacCap wird als wasserfeuchter Partikel erhalten.

### Beispiel 2

Es werden 3 L Lewatit S 8227 der Firma Lanxess auf einer Fritte mit der Porosität 3 mit 15 L vollentsalztem Wasser gewaschen. Es werden dann 2270 g feuchter Ionenaustauscher in einen Vakuumschaufeltrockner VT 5 der Firma Lödige eingewogen. Der Ionenaustauscher wird bei einer Manteltemperatur von 80°C und einem Druck von 30 mbar und einer Drehzahl von 57 Upm für 2 h getrocknet. Nach der Trocknung werden 915 g getrockneter Ionenaustauscher zurück in den Vakuumschaufeltrockner VT 5 gefüllt. Die Manteltemperatur wird auf 4°C eingestellt und wenn die Produkttemperatur unter 20°C liegt, werden innerhalb von 15 Minuten 600 ml vollentsalztes Wasser in dem Mischer, der bei einer Drehzahl von 180 Upm betrieben wird, mittels einer Schlauchpumpe gefördert. Für die Beschichtung werden 227 g Polyvinylamin Lösung (Polymergehalt 10 %) Lot: PC 18007 und 227 g vollentsalztes Wasser in einem Gefäß eingewogen. Als Vernetzer werden 9,20 g Ethylen-Glykol-Di-Glycidyl-ether (EGDGE) [2224-15-9] in einem anderen Gefäß eingewogen. Der Vernetzer wird zu der Polymerlösung gegeben und intensiv gemischt. Dann wird die Mischung innerhalb von 5 min mit einer Schlauchpumpe in den Lödige Mischer gefördert. Die Drehzahl des Mischers wird dabei auf 240 Upm eingestellt und die Manteltemperatur wird bei 4°C belassen. Nach der Zugabe wurde noch für 15 min bei 240 Upm gemischt. Dann wird die Manteltemperatur am Trockner auf 80°C eingestellt und die Drehzahl auf 120 Upm heruntergeregelt. Anschließend werden die Partikel wieder auf Raumtemperatur heruntergekühlt und werden dann auf geeignete Filternutsche überführt und mit folgenden Lösungsmitteln gewaschen:3 BV 0,1 M NaOH, 3 BV VE-Wasser, 3 BV 0,1 M HCl, 6 BV Wasser. Das Produkt BacCap wird als wasserfeuchter Partikel erhalten.

### Beispiel 3

500 g Trägermaterial sulfoniertes Polystyrol PRC 15035 (mittlere Porengröße 450 Ä, mittlere Partikelgröße 500 um) mit einer Wasseraufnahmekapazität von 1,35 ml/g werden direkt in einem Pflugscharmischer VT5 der Firma Lödige gesaugt. Die Produkttemperatur im Trockner wird auf 10 °C eingestellt. Der Mischer wird mit 180 Umdrehungen pro Minute betrieben. Nachdem die Produkttemperatur in der Mischtrommel 10°C erreicht hat, werden 225 g auf 10°C gekühlte nicht entsalzte Polyvinylaminlösung Lot.: PC 16012 (Polymergehalt 12%), 20 g Ethylen-Glykol-Di-Glycidyl-ether (EGDGE) CAS-Nr. [2224-15-9] und 430 g VE Wasser in ein Gefäß eingewogen. Die Mischung wird innerhalb von 10 min in dem Mischer gegeben und für 1 h bei 10°C gemischt. Anschließend wird das Polymeradsorbat bei 65°C und vernetzt. Das Produkt wird dann auf Raumtemperatur heruntergekühlt. Die Partikel werden dann auf geeignete Filternutsche überführt und mit folgenden Lösungsmitteln gewaschen: 3 BV 1 M NaOH, 3 BV VE-Wasser, 3 BV 2 M HCl, 3 BV Wasser, 6 BV 1 M NaOH, 6 BV VE-Wasser. Es werden 1297 g Produkt wird als wasserfeuchter Partikel erhalten. Anionenkapazität (AIK): 471 µmol/g.

### Beispiel 4

Vorschrift zur Herstellung eines porösen Partikeln eines vernetzten Polymers mit 100 um Partikelgröße (Batch: BV 18007): 1. Herstellung Polymeradsorbat: 750 g Trägermaterial Kieselgel (AGC Si-Tech Co. M.S Gel D-200-100 Lot.: 164M00711) werden direkt in einem Pflugscharmischer VT5 der Firma Lödige gefördert. Die Produkttemperatur wird auf 10°C eingestellt. Der Mischer wird mit 180 Umdrehungen pro Minute betrieben. Nach dem die Produkttemperatur in der Mischtrommel 10°C erreicht hat, werden 1125 g auf 10°C gekühlte nicht entsalzte Polyvinylaminlösung Lot.: PC 18007 (Polymergehalt 10%) in ein Gefäß eingewogen und mit 23,2 g Ethylen-Glykol-Di-Glycidyl-ether (EGDGE) CAS-Nr. [2224-15-9] versetzt. Die Mischung wird innerhalb von 10 min in dem Mischer gegeben und für 1 h bei 10°C gemischt. Anschließend wird das Polymeradsorbat bei 80°C und 50 mbar getrocknet (ca. 2 h). Das beschichtete Kieselgel wurde dann auf 10°C heruntergekühlt. Für die 2. Beschichtung wurden 750 g auf 10°C gekühlte Polymerlösung PC 18007 (Polymergehalt 10%) in ein Gefäß eingewogen und mit 15 g Ethylen-Glykol-Di-Glycidyl-ether (EGDGE) CAS-Nr. [2224-15-9] versetzt. Die Polymerlösung wurde innerhalb von 5 min in die Mischtrommel gefüllt. Das Polymeradsorbat wurde für 30 min bei 10°C gemischt. Anschließend wurde die Temperatur im Lödige Mischer wieder auf 65°C für 1 h erhöht. Das Polymeradsorbat wurde mit 3 L VE- Wasser versetzt. Diese Suspension wird für die Vernetzung benutzt. Das in Wasser suspendierte beschichtete Kieselgel wird in einen 10 1 Glasreaktor mit automatischer Temperierung überführt. Die Suspension wird gerührt und auf 80°C erhitzt. Anschließend werden 317 g Epichlorhydrin CAS-Nr. [106-89-8] innerhalb von 20 min zugegeben, sodass die Temperatur im Reaktor 85°C nicht überschreitet. Dann werden innerhalb von 20 Minuten 211 g 1,2-Diaminoethan [107-15-3] zu getropft. Dann erfolgt die zweite Zugabe von 317 g Epichlorhydrin CAS-Nr. [106-89-8] innerhalb von 20 Minuten gefolgt von nochmal 211 g 1,2-Diaminoethan CAS-Nr. [107-15-3]. Zum Schluss werden abschließend 317 g Epichlorhydrin CAS-Nr. [106-89-8] zugegeben und die Reaktion für 1 h bei 85°C gerührt. Die Reaktionsmischung wird dann auf 25°C abgekühlt, und es werden 1500 ml 50% NaOH zugesetzt und Reaktionsmischung wird für 12 Stunden gerührt. Die Templatpartikel werden dann auf geeignete Filternutsche überführt und mit folgenden Lösungsmitteln gewaschen: 3 BV 1 M NaOH, 3 BV VE-Wasser, 3 BV 2 M HCl, 3 BV Wasser, 6 BV 1 NaOH und 6 BV VE-Wasser.

Das Produkt wird als feuchter Filterkuchen erhalten.

### Beispiel 5

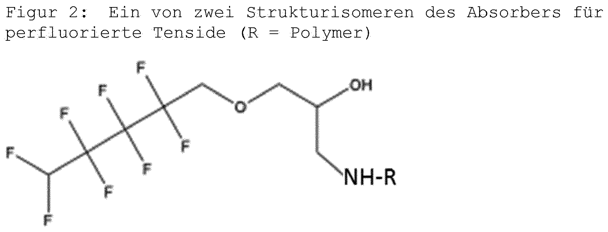

Ein Absorberharz, hergestellt gemäß Beispiel 1, Beispiel 3 oder Beispiel 4, wird in einem Lösungsmittel, z. B DMF suspendiert. Anschließend werden 110 mol% von Glycidyl-2,2,3,3,4,4,5,5-octafluoropentyl-ether (bezogen auf die Aminogruppen des Ausgangsharzens, Figur 2) zu der gerührten Suspension des Harzes gegeben und die Suspension für 12 h bei 70°C gerührt. Anschließend wird die Suspension mit auf einer Spritze mit folgenden Lösungsmitteln gespült: 3 BV DMF, 3 BV n-Heptan, 3 BV 1 M HCl in DMF, 3 BV 1 M NaOH in DMF und 3 BV DMF. Dann wird wieder ein 50%ige Suspension in DMF hergestellt. Zu dieser Suspension werden in einem zweiten Reaktionsschritt wieder 110 mol% Glycidyl-2,2,3,3,4,4,5,5-octafluoropentyl-ether gegeben. Die Reaktionsmischung wird anschließend für 12 h bei 70°C gerührt. Anschließend wird das Absorberharz mit folgenden Lösungsmitteln gespült: 3 BV DMF, 3 BV n-Heptan, 3 BV 1 M HCl in DMF, 3 BV 1 M NaOH in DMF und 3 BV MeOH. Das Absorberharz kann dann bis zur Gewichtskonstanz im Vakuum getrocknet werden.

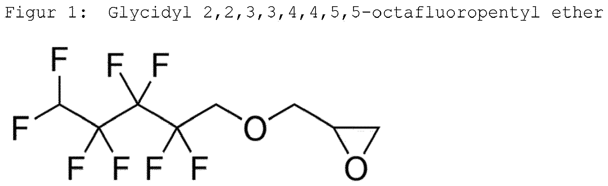

Die Struktur des Absorberharzes ist in Figur 1 gezeigt.

### Beispiel 6

Das wie in Beispiel 5 hergestellte Absorberharz, Aktivkohle (100Ä, 45 um) und kommerzielles C-18 Chromatographiegel (Kromasil, C18, 100 Ä, 10 um) wird in je eine HPLC-Säule des Formats 33,5 x 4 mm gefüllt, verschlossen und einer Frontalanalyse unterzogen. Dazu wird eine Lösung von 100 ppm Perfluoroctansäue in Wasser hergestellt und durch die Säule bis zur Erschöpfung der Kapazität gepumpt. Der Durchbruch wird UV-spektrometrisch bei 205 nm gemessen.

Die nachfolgende Tabelle (Tabelle) gibt die auf den drei Absorbern gemessenen Absorberkapazitäten für Perfluoroctansäue an:

**Tabelle 1 Perfluor-Oktansäure Kapazität des perfluor-Tensidselektiven instrAction Gels ND 150201 im Vergleich zu kommerziellen Absorbern**

| | | | |
|---|---|---|---|
| **ME 15036** | | | |
| Column | **PV 150778** | **PV 150775** | **PV 150772** |
| **Resin Batch** | **EP 14433** | **EP 15010** | **ND 150201** |

| **Perfluor-Oktansäure** | **Kromasil C18** | **Activated Charcoal** | **modified instrAction resin** |
|---|---|---|---|
| **Up-Take [mg/ml Phase]** | 0 | 64 | 220 |

Wie die Tabelle 1 zeigt, ist die Absorberkapazität für Perfluoroctansäue auf der instrAction Phase (Batch No. ND 150201) ca. 3-4 x größer als auf der Aktivkohle. Kommerzielles RP18 Gel zeigt keine Absorption von Perfluoroctansäure.

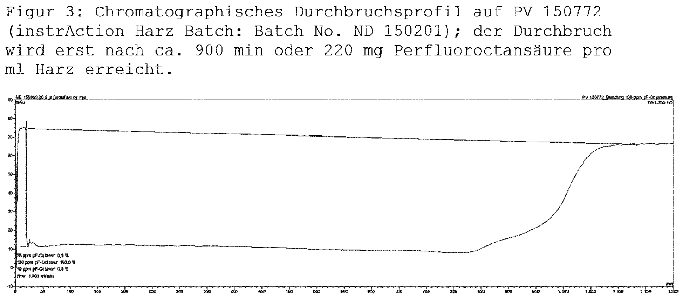

Die Durchbruchskurve der Frontalanalyse ist in Figur 3 gezeigt.

### Figurenverzeichnis:

Figur 1: Glycidyl 2,2,3,3,4,4,5,5-octafluoropentylether
Figur 2: Ein von zwei Strukturisomeren des Absorbers für perfluorierte Tenside (R = Polymer)
Figur 3: Chromatographisches Durchbruchsprofil auf PV 150772 (instrAction Harz Batch: Batch No. ND 150201); der Durchbruch wird erst nach ca. 900 min oder 220 mg Perfluoroctansäure pro ml Harz erreicht.

## Patentansprüche

**1.** Verfahren zur Herstellung von Partikeln zur Bindung perfluorierter Tenside, wobei das Verfahren die folgenden Schritte umfasst:
(a) Bereitstellen einer wässrigen Suspension enthaltend ein Polyamin, einen Vernetzer und ein poröses organisches oder anorganisches Trägermaterial in Partikelform bei einer Temperatur kleiner oder gleich 10°C in einem Mischer zum Beschichten des Trägermaterials mit dem Polyamin;
(b) Vernetzen des Polyamins in den Poren des Trägermaterials und gleichzeitiges Entfernen von Wasser.

**2.** Verfahren nach Anspruch 1, wobei der Schritt a) und b) wenigstens einmal wiederholt wird.

**3.** Verfahren nach einem der Ansprüche 1 oder 2, wobei das Vernetzen in einem Rührreaktor erfolgt.

**4.** Verfahren nach einem der Ansprüche 1 bis 3, wobei das Polyamin im nicht-entsalzten Zustand eingesetzt wird.

**5.** Verfahren nach einem der Ansprüche 1 bis 4, wobei das organische Trägermaterial ausgewählt ist aus Polystyrol, Polymethacrylat oder Polyacrylat.

**5.** Verfahren nach einem der Ansprüche 1 bis 4 wobei das organische Trägermaterial ein Polystyrol ist.

**6.** Verfahren nach einem der Ansprüche 1 bis 5, wobei das organische Trägermaterial ein starker oder ein schwacher Anionenaustauscher ist, der nur auf seiner äußeren Oberfläche mit dem Polyamin beschichtet ist.

**8.** Verfahren nach den Ansprüchen 1 bis 7 wobei das Trägermaterial ein anorganisches Polymer, ausgewählt aus Kieselgel oder Hydroxyapatit, ist.

**9.** Verfahren nach einem der Ansprüche 1 bis 8, wobei das Polyamin ein Polyvinylamin ist.

**10.** Verfahren nach einem der Ansprüche 1 bis 9, wobei das Polyamin in den Poren und/oder auf der Oberfläche des beschichteten Partikels mit Glycidyl-2,2,3,3,4,4,5,5-octafluoropentylether 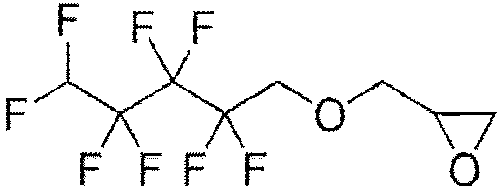 oder Perfluoroctansulfonsäure in der Seitenkette modifiziert wird.

**11.** Poröse Partikel, hergestellt nach einem Verfahren nach einem der Ansprüche 1 bis 10.

**12.** Poröse Partikel nach Anspruch 11, wobei die porösen Partikel einen Substituenten 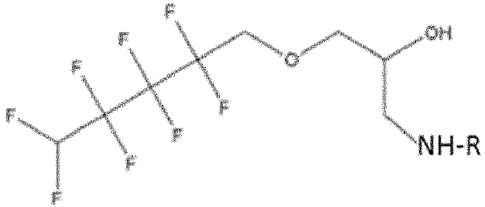 oder Perfluoroctansulfonsäure umfassen, wobei R das Polymer Polyvinylamin bedeutet.

**13.** Verwendung der porösen Partikel nach Anspruch 11 oder 12 oder hergestellt nach einem Verfahren nach einem der Ansprüche 1 bis 10 zur Bindung oder Entfernung von perfluorierten Tensiden aus Trinkwasser.
